(19) Europäisches Patentamt
European Patent Office
Office européen des brevets

(11) Veröffentlichungsnummer: **0 412 508 B1**

(12) **EUROPÄISCHE PATENTSCHRIFT**

(45) Veröffentlichungstag der Patentschrift: **05.07.95**

(51) Int. Cl.$^6$: **C07K 7/16**, A61K 38/22

(21) Anmeldenummer: **90115171.2**

(22) Anmeldetag: **07.08.90**

(54) **Vasopressin-Analoga,ihre Herstellung und ihre pharmazeutische Verwendung.**

(30) Priorität: **07.08.89 CS 4705/89**
**18.08.89 CS 4860/89**

(43) Veröffentlichungstag der Anmeldung:
**13.02.91 Patentblatt 91/07**

(45) Bekanntmachung des Hinweises auf die
Patenterteilung:
**05.07.95 Patentblatt 95/27**

(84) Benannte Vertragsstaaten:
**AT BE DE DK FR GB NL SE**

(56) Entgegenhaltungen:
**EP-A- 0 182 626**
**BE-A- 667 456**

**COLLECTION CZECHOSLOV. CHEM. COM-**
**MUN., Band 40, 1975, Seiten 905-912, Pra-**
**gue,CZ; M. ZAORAL et al.: "Preparation of**
**[1-beta-mercaptopropionic acid, 8-homoargi-**
**nine]-, and [1-beta-mercaptopropionic acid,**
**8-D-homoarginine]-vasopressin"**

(73) Patentinhaber: **CESKOSLOVENSKA AKADEMIE**
**VED**
**Narodni 3**
**Praha 1 (CS)**

(72) Erfinder: **Prochazka, Zdenko**
**Faltanova 564**
**Praha 4 (CS)**
Erfinder: **Blaha, Ivo**
**Lumieru 3**
**Praha 5 (CS)**
Erfinder: **Zertova, Miroslava**
**Hlavatého 618**
**Praha 4 (CS)**
Erfinder: **Slaninova, Jirina**
**Naf Lomem**
**Praha 4 (CS)**
Erfinder: **Velek, Jiri**
**Renvierova 619**
**Praha 5 (CS)**
Erfinder: **Skopkova, Jana**
**Juarezova 11**
**Praha 6 (CS)**
Erfinder: **Lebl, Michal**
**Rimska 36**
**Praha 2 (CS)**

COLLECTION CZECHOSLOV. CHEM. COM-MUN., Band 44, 1979, Seiten 1642-1644, Praque,CZ; V. KRCHNAK et al.: "Effect of methylation of the hydroxyl group of tyrosinein [1,beta-mercaptopropionic acid, 8-D-argini-ne]-vasopressin on its biologicaleffects"

PEPTIDES, PROCEEDINGS OF THE 9TH AMERICAN PEPTIDE SYMPOSIUM 1975, Ed. Deber etal., Seiten 615-618, Pierce Chemical Co., Rockford, III, US; N.C.F. YIM et al.:"The synthesis and structure-activity studies of vasopressin antagonistsmodified at positions one and two"

Erfinder: **Barth, Tomislav**
**Gottwaldova 883**
**Roztoky u Prahy (CS)**
Erfinder: **Maletinska, Lenka**
**Navigatoru 606**
**Praha 6 (CS)**
Erfinder: **Vilhardt, Hans**
**Tibberup allé 79**
**Espergarde (DK)**

(74) Vertreter: **Patentanwälte Beetz - Sieg-fried Schmitt-Fumian - Mayr**
**Steinsdorfstrasse 10**
**D-80538 München (DE)**

**Beschreibung**

Die Erfindung betrifft Analoga des 8-D-Homoarginin-Vasopressins, ein Verfahren zu ihrer Herstellung und ihre Verwendung in pharmazeutischen Mitteln.

Der Herstellung von Vasopressin-Derivaten, bei denen das Arginin in der 8-Position durch D-Homoarginin und das Tyrosin in der 2-Position durch nicht natürliche Aminosäuren ersetzt sind, wurde bereits in vielen Laboratorien großes Interesse gewidmet (Zaoral et al., Collection Czechoslovak. Chem. Commun. 31, 310 (1966); 31, 90 (1966); 32, 1250 (1967); 35, 1716 (1970); 37, 3350 (1972); 40, 905 (1975); 41, 2088 (1976); Huguenin und Boissonnas, Helv. Chim. Acta, 46, 1669 (1963); Lindberg et al., J. Med. Chem. 17, 781 (1974); 15, 629 (1972), Lindberg Int. J. Peptide Protein Res. 7, 395 (1975); Lindeberg et al., Int. J. Peptide Prot. Res. 8, 193 (1976); Bodanszky und Lindberg, J. Med. Chem. 14, 1197 (1971)). Die veröffentlichten Ergebnisse weisen auf die Veränderung einiger biologischer Wirkungen in Abhängigkeit vom Ersatz von L-Aminosäuren durch die entsprechenden D-Aminosäuren sowie von der Verschiebung der Position der positiv geladenen funktionalen Gruppe und der Seitenkette der Aminosäure in Position 8 der Peptidkette. Diese Verschiebung in der L-Reihe der Substituenten beeinflußt auch die Zugänglichkeit der durch die Carboxygruppe der basischen Aminosäure gebildeten Peptidbindung für eine Spaltung mit Trypsin (Dimeli, Barth, Collection Czechoslov. Chem. Commun. 44, 2451 (1979)).

Die Vergrößerung der Entfernung der Guanidinogruppe in der Vasopressinreihe (Homoargininreihe) läßt die antidjuretische Aktivität mehr oder weniger unverändert (Lindberg et al., J. Med. Chem. 17, 781, 1974); in der Desaminoreihe wurde bereits eine relative Herabsetzung festgestellt (Lindberg et al., J. med. Chem. 17, 781, 1974; Skopková et al., Collection Czechoslov. Chem. Commun. 46, 1850, 1981). Der Ersatz des L-Homoarginins durch die D-Form in der Desaminoreihe (Zaoral und Brtnik, Collection Czechoslov. Chem. Commun. 40, 905, 1975) ruft eine Herabsetzung der antidiuretischen Wirkung um mehr als eine Größenordnung hervor.

Der kombinierte Ersatz der Aminosäuren in den Positionen 2 und 8 der Peptidkette des 8-L-Arginin-Vasopressins ist eine der produktivsten Methoden der qualitativen und quantitativen Veränderung der biologischen Eigenschaften dieses Peptidtyps (K. Jost et al., Handbook of Neurohypophyseal Hormone Analogs, CRC Press, Boca Raton, U.S.A., 1987). Eine weitere Möglichkeit zur Abänderung bietet die freie Aminogruppe am Cystein in Position 1 des Peptids, vor allem im Sinne einer Änderung der Wirkungsdauer.

Mit dem Ziel, spezifisch wirksamere Inhibitoren der neurohypophysären Hormone zu gewinnen, wurden Analoga des Vasopressins und Desaminovasopressins mit D-Homoarginin in der 8-Position, welche in Position 2 weiter modifiziert wurden, synthetisiert.

Der Erfindung liegt entsprechend die Aufgabe zugrunde, Analoga des Vasopressins und Desaminovasopressins, ein Verfahren zu ihrer Herstellung sowie ihre pharmazeutische Verwendung anzugeben.

Die Aufgabe wird anspruchsgemäß gelöst. Die abhängigen Ansprüche betreffen vorteilhafte Ausführungsformen.

Die erfindungsgemäßen Analoga des 8-D-Vasopressins besitzen die allgemeine Formel

$$R-X-Phe-Gln-Asn-Cys-Pro-D-Har-Gly-NH_2,$$

in der bedeuten:

X    L-O-Methyltyrosin (Verbindungen der Formeln I, VI),

L-p-Ethylphenylalanin (Verbindungen der Formeln II, VII),

D-p-Ethylphenylalanin (Verbindungen der Formeln III, VIII),

L-p-Methylphenylalanin (Verbindungen der Formeln IV, IX) oder

D-p-Methylphenylalanin (Verbindungen der Formeln V, X)

und

R    Cystein oder $\beta$-Mercaptopropionsäure.

Das Wesen des erfindungsgemäßen Verfahrens zur Herstellung dieser Verbindungen liegt darin, daß durch Festphasensynthese aus den Hydroxybenzotriazolestern der durch t-Butyloxycarbonylgruppen geschützten Aminosäuren eine lineare Kette aufgebaut wird, die dann durch Einwirkung von flüssigem Fluorwasserstoff von der festen Phase unter gleichzeitiger Abspaltung der Schutzgruppen aus den Seitenketten freigesetzt wird, wonach die Cyclisierung mit Kaliumhexacyanoferrat(III) durchgeführt wird und die Peptide, die in der 2-Position der Peptidkette L- und D-Isomere der Aminosäuren enthalten, durch Hochdruckflüssigkeitschromatographie getrennt werden.

Die angeführten Verbindungen sind befähigt, die uterotonische Wirkung der neurohypophysären Hormone zu unterdrücken. Sie können daher z.B. in der Humanmedizin als Mittel zur Blockierung der vorzeitigen Geburt, die durch das endogene Oxytocin oder Vasopressin hervorgerufen wird, verwendet werden. Die Verbindungen VIII und X gehören zu den wirksamsten Inhibitoren, die bisher aus der Vasopressinreihe hergestellt wurden, wie aus der nachstehenden Tabelle hervorgeht.

Biologische Wirksamkeit erfindungsgemäßer Verbindungen im Vergleich mit den bekannten Vasopressin-Analoga $[D\text{-}Har^8]$-Vasopressin und $[Mpr^1, D\text{-}Har^8]$-Vasopressin

| Verbindung | Bezeichnung | Uterotonische Wirksamkeit [IU/mg] | Pressorische Wirksamkeit | Antidiuretische Wirksamkeit (% dDAVP) |
|---|---|---|---|---|
| $[D\text{-}Har^8]$Vasopressin | | 0,9 | (nicht bestimmt) | ~ 1 |
| $[Mpr^1, D\text{-}Har^8]$Vasopressin | | 0,8 | 0,28 IU/mg | ~ 5 |
| $[D\text{-}Me\text{-}Tyr^2, D\text{-}Har^8]$- | I | $pA_2 = 7,7$ | 0,04 IU/mg | (nicht bestimmt) |
| $[L\text{-}p\text{-}EtPhe^2, D\text{-}Har^8]$- | II | $pA_2 = 7,40$ | $pA_2 = 6,5$ | < 0,1 |
| $[D\text{-}pEtPhe^2, D\text{-}Har^8]$- | III | $pA_2 = 8,15$ | $pA_2 = 6,5$ | < 0,1 |
| $[L\text{-}pMePhe^2, D\text{-}Har^8]$- | IV | $pA_2 = 6,85$ | 0,04 IU/mg | < 0,1 |
| $[D\text{-}p MePhe^2, D\text{-}Har^8]$- | V | $pA_2 = 6,85$ | 0,04 IU/mg | < 0,1 |
| $[Mpr^1, OMeTyr^2, D\text{-}Har^8]$-Vasopressin | VI | $pA_2 = 8,1$ | a | (nicht bestimmt) |
| $[Mpr^1, L\text{-}p\text{-}EtPhe^2, D\text{-}Har^8]$-Vasopressin | VII | $pA_2 = 8,0$ | $pA_2 = 6,2$ | < 1 |
| $[Mpr^1, D\text{-}p\text{-}EtPhe^2, D\text{-}Har^8]$-Vasopressin | VIII | $pA_2 = 8,63$ | $pA_2 = 6,35$ | < 1 |
| $[Mpr^1, L\text{-}p\text{-}MePhe^2, D\text{-}Har^8]$-Vasopressin | IX | $pA_2 = 8,58$ | $pA_2 = 6,2$ | < 1 |
| $[Mpr^1, D\text{-}p\text{-}MePhe^2, D\text{-}Har^8]$-Vasopressin | X | $pA_2 = 6,58$ | a | < 1 |

Als geeignetes Derivat des D-Homoarginins wurde für die Synthese aller Analoga das N-$\alpha$-tert-Butyloxycarbonyl-NG-nitrohomoarginin verwendet; der p-Methylphenylalanin- und der p-Ethylphenylalaninrest werden in der Form eines Gemisches der L- und D-Aminosäuren eingeführt.

Die Synthese der Verbindungen der Formel I bis X wurde an fester Phase unter Verwendung eines Benzhydrylaminharzes durchgeführt. Zum Schutz der $\alpha$-Aminogruppen wurde die t-Butyloxycarbonylgruppe (BOC) verwendet; zur Blockierung der Gruppen in den Seitenketten wurden die Nitrogruppe (D-Homoarginin), 4-Methylbenzyl (Cystein), Benzyloxycarbonyl (Tyrosin) und Benzyl ($\beta$-Mercaptopropionsäure) verwendet. Die Kondensation der geschützten Aminosäuren wurde in Dimethylformamid unter Anwendung der entsprechenden aktiven Hydroxybenzotriazolester durchgeführt. Die Schutzgruppen in den Seitenketten wurde gleichzeitig mit der Ablösung der Peptidkette vom Träger durch Fluorwasserstoff abgespalten. Die Oxidation der SH-Gruppe und die darauffolgende Cyclisierung wurden mit einer Kaliumhexacyanoferrat(III)-Lösung durchgeführt. Die Stoffe wurden gereinigt und die Peptide mit D- und L-Aminosäuren in Position 2 durch Kochdruckflüssigkeitschromatographie(HPLC) getrennt. Für die Herstellung von Diastereomerengemischen der Analoga mit D,L-p-Ethylphenylalanin oder D,L-p-Methylphenylalanin wurden nur 1.1-Äquivalent des t-Butyloxycarbonyl-D,L-p-Ethylalanins bzw. t-Butyloxycarbonyl-D,L-p-methylphenylalanins verwendet.

Die gewonnenen Verbindungen wurden durch Dünnschichtchromatographie (TLC) an Kieselsäureplatten (Silufol, Kavalier, CS) unter Verwendung der folgenden Systeme charakterisiert:

2-Butylalkohol-98 %ige Ameisensäure-Wasser (10:3:8) oder

1-Butylalkohol-Essigsäure-Pyridin-Wasser (15:3:10:6).

Die Elektrophorese wurde in 1M Essigsäure oder in PyridinAcetat-Puffer (pH 5,7) auf Whatman 3 MM-Papier während einer Dauer von 1 Stunde bei einem Potentialgradienten von 20 V/cm durchgeführt. Die Aminosäureanalyse wurde mit einem Aminosäureanalysator (T 339 oder D-500, Durrum Corp., USA) durchgeführt. Die analytische HPLC wurde auf einer Kolonne mit Separon S IX-C 18- oder Vydac Z 18 TP 5-Füllung vorgenommen, die präparative HPLC mit einem modularen Aufbau (Knauer) und einer mit Sepharon SGX-C18 gefüllten Kolonne.

Nachstehend sind folgende Abkürzungen verwendet:

| | |
|---|---|
| HPLC | Hochdruckflüssigkeitschromatographie |
| TLC | Dünnschichtchromatographie |
| BOC | t-Butyloxycarbonyl |
| Mpr | $\beta$-Mercaptopropionsäure |
| Bzl | Benzyl |
| TFA | Trifluoressigsäure |
| MeOH | Methanol |
| Bt | Benzotriazol. |

Der Zyklus zur Bindung der geschützten Aminosäure beim Aufbau der Peptidkette am Träger kann durch das folgende Schema beschrieben werden:

1. Abspaltung der BOC-Gruppe mit 40 ml 50 %iger Trifluoressigsäure in Dichlormethan mit 2 % Anisol während einer Dauer von 2 min, wiederholt nach 30 min;

2. Waschen mit Dichlormethan (3x40 ml; Dauer eines Waschvorgangs 30 s);

3. Waschen mit 30-%igem Dioxan in Dichlormethan (3x40 ml; Dauer eines Waschvorgangs 30 s);

4. Waschen mit Dichlormethan (3x40 ml, Dauer eines Waschvorgangs 30 s);

5. Die Neutralisation mit 10 %igem Triethylamin (40 ml) und Dichlormethan (40 ml; Zyklus 2 min);

6. Waschen mit Dichlormethan (2x40 ml, Zyklus 30 s);

7. Die Neutralisation durch Zugabe von 40 ml 10 %igem Triethylamin in 40 ml Dichlormethan (Zyklus 2 min);

8. Waschen mit Dichlormethan (6x40 ml, Zyklus 30 s);

9. Zugabe des Hydroxybenzotriazolesters der BOC-geschützten Aminosäuren in Dichlormethan bis zu negativem Ninhydrintest (30-120 min).

10. Waschen mit 50-%igem Ethanol in Dichlormethan (3x40 ml, Zyklus 30 s);

11. Waschen mit Dichlormethan (3x15 ml; Zyklus 1 min).

Das Benzhydrylharz (4,46 g; 0,56 mmol/g) wurde in Dichlormethan verrührt und nach dem Waschen mit 5 %igem Triethylamin in Dichlormethan und Dimethylformamid mit einem 3-fachen molaren Überschuß von BOC-Gly-OBt vermischt. Die Reaktion wurde nach 30 min unterbrochen und das Harz nacheinander mit Dimethylformamid (3x20 ml) und Dichlormethan (3x20 ml) gewaschen. Danach wurde nach dem Verfahren nach Beispiel 4 weitergearbeitet. Die BOC-Aminosäuren in der Form der aktiven Ester (in 3-fachem molarem Überschuß) wurden an die Aminogruppen der gebundenen Aminosäuren in folgender Reihenfolge angekuppelt: BOC-D-Har(NO$_2$)-OH, BOC-Pro-OH, BOC-Cys(4-Me-Bzl)-OH, BOC-Asn-OH, BOB-Gln-OH und BOC-Phe-OH. Im Falle der Bindung von Homoarginin, Cystein und Phenylalanin wurde zur Beschleunigung der Reaktion ein Katalysator (4-Dimethylaminopyridin, 50 mg) hinzugefügt.

Die biologische Wirksamkeit wurde an Ratten bestimmt: Die uterotonische (agonistische oder inhibitorische) Wirksamkeit wurde nach Holton (Holton, J. Brit. J. Pharmacol. 3, 328, 1948) in der Bearbeitung nach Munich (Endocrinology 66, 451, 1960) bestimmt; die inhibitorische Aktivität wurde ausgedrückt als pA2 (Eggena et al., J. Gen. Physiol. 56, 250, 1970), die pressorische Wirksamkeit wurde an despinalisierten Rattenmännchen nach Krejci et al., (Brit. J. Pharm. Chemother. 30, 497, 1967) bestimmt, die antidiuretische nach Burn (Burn et al., Biol. Stand., Oxford Univ. Press London 1950). Als Standard wurde für den antidiuretischen Test das [1-Desamino,8-D-Arginin]-Vasopressin (dDAVP) verwendet. Eine Übersicht der biologischen Wirksamkeiten ist der Tabelle 1 zu entnehmen.

Im folgenden wird die Herstellung der Verbindungen I bis X anhand von Beispielen erläutert.

Beispiel 1

Herstellung von [1-Mercaptopropionsäure,2-0-Methyltyrosin, 8-D-Homoarginin]-Vasopressin (Verbindung der Formel VI)

An das Harz mit gebundenem Heptapeptid (0,91 g; 0,33 mmol) wurden BO-Tyr(Me)OH und Mpr(Bzl)OH gekuppelt. Der Harz mit dem gebundenen Nonapeptid (1 g) wurde der Einwirkung von flüssigem Fluorwasserstoff (10 ml, 60 min, 0 °C in Gegen-wart von Anisol (1,5 ml)) ausgesetzt. Danach wurde der Fluorwasserstoff mit Stickstoff bei 0 °C während 30 min entfernt. Das Gemisch des freien Nonapeptids und des Harzes wurde mit Ether geschüttelt; nach dem Abfiltrieren wurde mit Ethylacetat gewaschen.

Das freie Peptid wurde in 20 %iger Essigsäure (100 ml) bei 40 °C gelöst und die Lösung nach dem Verdünnen mit Wasser lyophylisiert. Das Lyophilisat wurde in Wasser gelöst (300 ml) und der pH-Wert der Lösung auf 7,0 eingestellt. Zu der Lösung wurde 0,01 M Kaliumhexacyanoferrat(III) bis zur stabilen Gelbfärbung hinzugefügt. Nach Beendigung der Oxidation (30 min) wurde der pH-Wert mit Essigsäure auf 4,5 eingestellt. Die Lösung wurde nachher auf eine Amberlite CG-50 I-Säule aufgetragen, die Säule mit 0,25 %iger Essigsäure (150 ml) gewaschen und das Produkt mit 50 %iger Essigsäure (60 ml) eluiert. Das Produkt wurde lyophilisiert (47 mg) und durch HPLC gereinigt. Die Ausbeute betrug 9 mg. Aminosäureanalyse: Phe 0,87, Cys 0,91, Asp 1,01, Glu 0,98, Pro 0,96, Gly 1,00, Tyr(Me) 0,96, Har 0,92.

Beispiel 2

Herstellung von [1-Mercaptopropionsäure-2-p-Ethyl-D,L-phenylalanin,8-D-Homoarginin] -Vasopressin (Verbindungen der Formeln VII und VIII)

An das Harz mit gebundenem Heptapeptid (1,35 g; 0,6 mmol) wurden nach dem Schema 1,1 Äquivalent BOC-L,D-p-Ethylphenylalanin im Verlauf von 24 h und danach noch 1 Äquivalent in Gegenwart von Dimethylaminopyridin während 30 min gekuppelt; dann wurde an das Octapeptid Mpr(Bzl)OH nach dem angeführten Schema gebunden. Das Harz mit dem gebundenen Nonapeptid (1,4 g) wurde in Gegenwart von Anisol (1,5 ml) der Einwirkung von flüssigem Fluorwasserstoff (10 ml, 60 min, 0 °C) unterworfen. Danach wurde der HF mit Stickstoff bei 0 °C während 30 min entfernt. Das abgespaltene Nonapeptid wurde zusammen mit dem Harz mit Ether vermischt, abfiltriert, mit Ethylacetat gewaschen und in 20 %iger Essigsäure (100 ml) bei 40 °C aufgelöst; nach Zugabe von Wasser wurde die Lösung lyophilisiert. Das Lyophilisat wurde in Wasser (500 ml) gelöst und der pH-Wert des Lyophilisats mit Ammoniumhydroxid auf 7 eingestellt. Anschließend wurde zu der Lösung eine 0,01 M-Lösung von Kaliumhexacyanoferrat(III) bis zur stabilen Gelbfärbung hinzugegeben. Nach Beendigung der Oxidation (30 min) wurde der pH-Wert mit Essigsäure auf 4,5 eingestellt. Das Produkt wurde auf einer Amberlite CG-50 I-Säule adsorbiert. Die Säule wurde mit 0,25 %iger Essigsäure gewaschen; das Produkt wurde mit 50 %iger Essigsäure (60 ml) eluiert. Durch Lyophilisierung des Eluats wurden 41 mg Produkt gewonnen.

Trennung des Racemats [1-Mercaptopropionsäure-2-p-Ethyl-D,L-phenylalanin,8-D-Homoarginin]-Vasopressin (Verbindungen der Formeln IX und X).

Die präparative Flüssigkeitschromatographie wurde mit einer modularen Anordnung (bestehend aus Knauer HPLC-Programmer 50, Knauer HPLC-Pumpe 364 und Knauer-Detektor mit veränderlicher Wellenlänge) und mit einer Säule (250 x 16 mm) durchgeführt, die mit Sepharon SG-X-C18 (10 μm) gefüllt war. Das Peptid wurde in einer Menge von 5 mg eingespritzt und mit Methanol eluiert, wobei ein Konzentrationsgradient des Methanols angewandt wurde (Start 50 %; 1 %/min). Retentionszeit der L-Form 10,05 min, der D-Form 11,97 min. Das L-Derivat wurde in einer Menge von 10 mg, das D-Derivat in einer Menge von 8 mg gewonnen.

Aminosäureanalyse:
L-Form: EtPhe 0,80, Phe 1,03, Cys 0,93, Asp 0,95, Glu 1,12, Pro 0,98, Gly 1,00, Har 0,87;
D-Form: EtPhe 0,85, Phe 1,00, Cys 0,93, Asp 0,93, Glu 1,16, Pro 0,97, Gly 1,03, Har 0,87.

## Beispiel 3

Herstellung von [1-Mercaptopropionsäure,2-p-Methyl-L,D-phenylalanin,8-D-Homoarginin]-Vasopressin (Verbindungen der Formeln IX und X)

An dem Harz mit dem gebundenen Heptapeptid (1,35 g; 0,5 mmol) wurden nach dem Verfahren von Beispiel 7 BOC-L,D-p-MePhe-OH und Mpr(Bzl)OH angekuppelt. Die Peptide wurden wie in Beispiel 7 vom Harz abgespalten und isoliert. Die Ausbeute betrug 50 mg Peptid.

Trennung des Racemats [1-Mercaptopropionsäure,2-p-Methyl-L,D-phenylalanin,8-D-Homoarginin]-Vasopressin:

Die Racemattrennung wurde wie in Beispiel 2 durchgeführt.

Unter den angegebenen Bedingungen ist das Isomer mit L-p-Methylphenylalanin beweglicher (Retentionszeit 13,57 - 13,73 min) als die D-Form (Retentionszeit 15,72 - 15,75 min).

Es wurden 4,2 mg L-Derivat und 5,6 mg D-Derivat gewonnen.

Aminosäureanalyse:
L-Form: MePhe 0,9, Phe 1,03, Cys 1,05; Asp 0,97, Glu 1,13, Pro 0,99, Gly 1,05, Har 0,87;
D-Form: MePhe 0,87, Phe 1,0, Cys 1,06, Asp 0,95, Glu 1,10, Pro 1,00, Gly 1,06, Har 0,87.

## Beispiel 4

Herstellung von [2-O-Methyltyrosin,8-D-Homoarginin]-Vasopressin (Verbindung der Formel I)

Am Harz mit dem gebundenen Heptapeptid (1,7 g; 0,48 mmol) wurden nach der oben angegebenen allgemeinen Verfahrensweise BOC-Tyr(Me)OH und BOC-Cys(4-Me-Bzl)OH angekuppelt. Das Harz mit dem gebundenen Nonapeptid (1,8 g) wurde dann der Einwirkung von flüssigem HF (20 ml, 60 min, 0 °C) in Gegenwart von 1,0 ml Anisol und 1,2-Ethandiol (1 ml) ausgesetzt. Anschließend wurde der Fluorwasserstoff abgetrennt.

Das freigesetzte Nonapeptid wurde nach der Entfernung des HF zusammen mit dem Harz mit Ether vermischt, abfiltriert und mit Ethylacetat gewaschen. Das freie Peptid wurde nacheinander mit Essigsäure, 50 %iger Essigsäure sowie mit Wasser extrahiert und danach lyophilisiert (Ausbeute 0,69 g). Das Lyophilisat wurde in Wasser gelöst (700 ml); der pH-Wert der Lösung wurde mit 0,1 N Natriumhydroxid auf 7,0 eingestellt. Die Lösung wurde mit Kaliumhexacyanoferrat(III) (0,01 M) bis zur stabilen Gelbfärbung versetzt.

Während der Oxidation (20 min) wurde der pH-Wert mit NaOH-Lösung (0,1 N) auf 7,2 gehalten; danach wurde der pH-Wert mit Essigsäure auf 4,5 herabgesetzt.

Das Produkt wurde dann an einer Amberlite CG-50 I-Säule adsorbiert; die Säule wurde mit 0,25 %iger Essigsäure gewaschen. Das Produkt wurde mit 50 %iger Essigsäure (60 ml) eluiert, lyophilisiert (353 mg) und danach durch Säulenchromatographie (Füllmaterial Vydac 218 TP 5) unter Elution mit Methanol gereinigt (linearer Gradient von 20 bis 40 % Methanol (MeOH) - 0,05 % Trifluoressigsäure (TFA) gereinigt. Nach der Lyophilisation wurden 160 mg Produkt erhalten.

| Elementaranalyse für $C_{48}H_{67}N_{13}O_{12}S_2 \times 3TFA \times 2H_2O$: | | | |
|---|---|---|---|
| | C (%) | H (%) | N (%) |
| berechnet: | 43,26 | 5,18 | 14,01 |
| gefunden: | 42,92 | 4,92 | 14,31 |

Aminosäureanalyse:
Asp 1,00, Glu 1,00, Pro 0,75, Gly 0,99, Cys 1,8, Tyr(Me) 1,1, Phe 1,07, Har 1,05.

Beispiel 5

Herstellung von [2-p-Ethyl-L-phenylalanin,8-D-Homoarginin]-Vasopressin (Verbindung der Formel II)

Das Harz (1,35 g; 0,5 mmol) mit dem gebundenen Heptapeptid (Beispiel 4) wurde 24 h mit 1,1 Äquivalent BOC-L,D-Phe(p-Et)OH und anschließend 30 min mit 1 Äquivalent in Gegenwart von Dimethylaminopyridin und danach mit BOC-Cys(4-Bzl)OH umgesetzt. Nach Abspaltung der BOC-Schutzgruppen wurde das Harz mit Fluorwasserstoff (15 ml, 60 min, 0 °C) in Gegenwart von 2 ml Anisol behandelt.

Das erhaltene freie Nonapeptid wurde zusammen mit dem Harz nach Abtrennung des Fluorwasserstoffs mit Ether vermischt, abfiltriert und mit Ethylacetat gewaschen. Das freie Peptid wurde mit Essigsäure, 50 %iger Essigsäure und Wasser extrahiert und danach mit einer 0,1 M Natriumhydroxidlösung auf pH 7,0 neutralisiert. Die Lösung wurde mit 0,01 M Kaliumhexacyanoferrat(III) bis zur stabilen Gelbfärbung versetzt. Während der Oxidation (20 min) wurde der pH-Wert durch Zu-gabe von 0,1 M Natriumhydroxidlösung auf 7,2 gehalten; abschließend wurde der pH-Wert mit Essigsäure auf 4,5 eingestellt.

Die Peptidlösung wurde an einer Amberlite CG- 50 I-Säule adsorbiert. Die Säule wurde mit 0,25 %iger Essigsäure gewaschen. Das Produkt wurde mit 50 %iger Essigsäure eluiert, lyophilisiert (185 mg) und durch HPLC an einer mit Sepharon SGX-C 18 gefüllen Säule mit einem linearen Methanolgradienten (20 bis 70 % - 0,1 % TFA) gereinigt.

Der erste Peak (charakterisiert durch die Beweglichkeit k' = 2,2, MeOH - 0,05 % TFA (6:4) und k' = 7,47 MeOH - 0,05 % TFA (5:5) ) entspricht dem Vasopressin-Analogon mit p-Ethyl-L-phenylalanin in Position 2.

| Elementaranalyse für $C_{49}H_{71}O_{11}S_2x4TFAx2H_2O$ (1602,5): | | | |
|---|---|---|---|
| | C (%) | H (%) | N (%) |
| berechnet: | 42,79 | 4,97 | 13,11 |
| gefunden: | 42,85 | 5,05 | 13,37 |

Aminosäureanalyse:
Asp 0,99, Glu 1,00, Pro 1,12, Gly 1,01, Cys (bestimmt als Cysteinsäure) 2,12, 4-Et-Phe 0,78, Phe 1,02, Har 0,80.

Die Ausbeute betrug 18 mg.

Beispiel 6

Herstellung von [2-p-Ethyl-D-phenylalanin,8-D-Homoarginin]-Vasopressin (Verbindung der Formel III)

Die Verbindung wurde nach Beispiel 5 im Gemisch mit der Verbindung der Formel II hergestellt. Sie wurde von dieser durch HPLC an einer Säule mit Sepharon SGX-C 18 wie in Beispiel 4 beschrieben abgetrennt.

Unter Anwendung eines linearen Konzentrationsgradienten MeOH (20-70 %) - 0,05 % TFA wurde diese Verbindung im zweiten Peak eluiert, die durch folgende Parameter gekennzeichnet ist: k' = 3,60, MeOH - 0,05 % TFA (6:4), k' = 19,57 MeOH - 0,05 % TFA (5:5).

| Elementaranalyse für $C_{49}N_{15}H_{71}O_{11}x2TFA$ (1566,4): | | | |
|---|---|---|---|
| | C (%) | H (%) | N (%) |
| berechnet: | 43,71 | 4,83 | 13,41 |
| gefunden: | 43,74 | 5,16 | 13,52 |

Aminosäureanalyse: Asp 1,00, Glu 0,98, Pro 1,05, Gly 1,12, Cys (bestimmt als Cysteinsäure) 2,02, 4-Et-Phe 0,68, Phe 1,05, Har 0,85.

Beispiel 7

Herstellung von [2-p-Methyl-L-phenylalanin,8-D-Homoarginin]-Vasopressin (Verbindung der Formel IV)

Das Harz mit dem gebundenen Heptapeptid (3,4 g; 0,96 mmol) wurde nach der oben angegebenen allgemeinen Verfahrensweise mit 1,1 Äquivalent BOC-L,D-Phe(p-Me)OH 18 h und anschließend noch 4 h mit 0,5 Äquivalent umgesetzt.

Als weitere Aminosäure wurde BOC-Cys(4-Me-Bzl)OH angekuppelt.

Das Harz mit dem gebundenen geschützten Peptid wurde wie in Beispiel 5 weiterverarbeitet. Aus einer Amberlite CG-50 I-Säule wurden mit 50 %iger Essigsäure 614 mg Rohprodukt eluiert, das lyophilisiert und durch HPLC (Säule Vydac 218 TP 5) unter Elution mit einem linearen Konzentrationsgradienten MeOH (25-60 %) - 0,05 % TFA gereinigt wurde.

Zunächst wurden 40 mg [2-p-Methyl-L-phenylalanin,8-D-Homoarginin]-Vasopressin eluiert, das wie folgt charakterisiert ist: k' = 3,18 MeOH - 0,05 % TFA (55:45).

| Elementaranalyse für $C_{48}H_{67}N_{13}O_{12}S_2 x3TFAx2H_2O$ (1474,4): | | | |
|---|---|---|---|
| | C (%) | H (%) | N (%) |
| berechnet: | 43,99 | 5,20 | 14,25 |
| gefunden: | 44,19 | 4,96 | 14,41 |

Aminosäureanalyse: Asp 1,08, Glu 1,01, Pro 0,87, Gly 1,08, Cys (bestimmt als Cysteinsäure) 2,04, 4-Me-Phe 0,7, Phe 0,92, Har 1,01.

Beispiel 8

Herstellung von [2-p-Methyl-D-phenylalanin,8-D-Homoarginin]-Vasopressin(Verbindung der Formel V)

Die Verbindung wurde im Gemisch mit der Verbindung der Formel IV nach Beispiel 7 hergestellt und von dieser durch HPLC (Säule Vydac 218 TP 5) unter Elution mit einem linearen Konzentrationsgradienten MeOH (25-60 %) - 0,05 % TFA getrennt.

Die Verbindung der Formel V wurde als zweite eluiert (16,2 mg). Sie ist wie folgt charakterisiert: k' = 5,18, MeOH - 0,05 % TFA (55:45).

| Elementaranalyse für $C_{48}H_{67}N_{13}O_{12}S_2 x3,5TFAx1H_2O$ (1522,4): | | | |
|---|---|---|---|
| | C (%) | H (%) | N (%) |
| berechnet: | 43,39 | 5,00 | 13,80 |
| gefunden: | 43,24 | 4,78 | 14,13 |

Aminosäureanalyse: Asp 0,89, Glu 1,02, Pro 1,00, Gly 1,05, Cys (bestimmt als Cysteinsäure) 2,03, 4-Me-Phe 0,88, Phe 1,00, Har 1,08.

**Patentansprüche**

**1.** Analoga des 8-D-Homoarginin-Vasopressins der allgemeinen Formel

$$R-X-Phe-Gln-Asn-Cys-Pro-D-Har-Gly-NH_2,$$

in der bedeuten:

X    L-O-Methyltyrosin,
       L-p-Ethylphenylalanin,
       D-p-Ethylphenylalanin,
       L-p-Methylphenylalanin oder

D-p-Methylphenylalanin
und

R        Cystein oder $\beta$-Mercaptopropionsäure.

2. Verfahren zur Herstellung der 8-D-Homoarginin-Vasopressin-Analoga nach Anspruch 1, gekennzeichnet durch
- Aufbau der linearen Aminosäurekette durch Festphasensynthese aus den Hydroxybenzotriazolestern der mit t-Butyloxycarbonylgruppen geschützten Aminosäuren,
- Abspaltung der Aminosäurekette durch Einwirkung von flüssigem Fluorwasserstoff unter gleichzeitiger Abspaltung der Seitenketten-Schutzgruppen,
und
- Cyclisierung durch Einwirkung von Hexacyanoferrat(III)
sowie ggf.
- Trennung der Peptide, die D- und L-Isomere der Aminosäuren in 2-Position enthalten, durch Hochdruckflüssigkeitschromatographie.

3. Verfahren nach Anspruch 2, dadurch gekennzeichnet, daß als Festphase ein Benzhydrylaminharz verwendet wird.

4. Pharmazeutische Mittel, gekennzeichnet durch ein oder mehrere Analoga des 8-D-Homoarginin-Vasopressins nach Anspruch 1 als Wirkstoffe.

**Claims**

1. Analogues of 8-D-homoarginine-vasopressin of the general formula

$$\text{R-X-Phe-Glu-Asn-Cys-Pro-D-Har-Gly-NH}_2,$$

wherein,
X        is L-O-methyltyrosine,
L-p-ethylphenylalanine,
D-p-ethylphenylalanine,
L-p-methylphenylalanine or
D-p-methylphenylalanine,
and
R        is cysteine or $\beta$-mercaptopropionic acid.

2. Method for the preparation of the 8-D-homoarginine-vasopressin analogues according to claim 1, characterized by
- synthesis of the linear aminoacid chain through solid phase synthesis from the hydroxybenzotriazole esters of the amino acids protected with t-butoxycarbonyl groups,
- separation of the aminoacid chain through action of liquid hydrogen fluoride with simultaneous chleavage of the side-chain protective groups,
and
- cyclization through action of hexacyanoferrate(III),
and optionally
- separation of the peptides containing the D- and L-isomers of the aminoacids in position 2 through HPLC.

3. The method according to claim 2, characterized in that a benzhydrylamine resin is used as solid phase.

4. Pharmaceutical compositions, characterized by one or several analogues of 8-D-homoarginine-vasopressin according to claim 1 as active ingredients.

**Revendications**

1. Analogues de la 8-D-homoarginine-vasopressine de la formule générale

$$\underline{R-X-Phe-Glu-Asn-Cys}-Pro-D-Har-Gly-NH_2,$$

dans laquelle

X     signifie L-O-méthyltyrosine,
      L-p-éthylphenylalanine,
      D-p-éthylphenylalanine,
      L-p-méthylphenylalanine ou
      D-p-méthylphenylalanine,
      et
R     désigne cystéine ou l'acide $\beta$-mercaptopropionique.

2. Procédé pour la préparation des analogues de la 8-D-homoarginine-vasopressine selon la revendication 1, charactérisé par
   - synthèse de la chaîne linéaire des aminoacides par synthèse en phase solide en utilisant les esters hydroxybenzotriazoliques des aminoacides protégées par des groupes t-butoxycarbonyle,
   - séparation de la chaîne des aminoacides par action de fluorure d'hydrogéne liquide avec clivage simultané des groupes protecteurs des chaînes latérales et
   - cyclisation par action d'hexacyanoferrate(III), et éventuellement
   - séparation de peptides contenant les isoméres D et L des aminoacides en position 2 par HPLC.

3. Procédé selon la revendication 1, caractérisé en ce qu'on utilise une résine à la base de la benzhydrylamine en tant que phase solide.

4. Composition pharmaceutiques, caractérisées par un ou plusieurs analogues de la 8-D-homoarginine-vasopressine selon la revendication 1 en tant que matières actives.